# EUROPEAN PATENT APPLICATION

(11) **EP 4 461 321 A1**
(43) Date of publication of application: **13.11.2024**
(21) Application number: 23382430.9
(22) Date of filing: 09.05.2023
(51) Int. Cl.: A61L 9/12, B32B 27/00, B65D 43/00

(54) **DEVICE FOR DIFFUSING VOLATILE SUBSTANCES AND METHOD FOR MANUFACTURING SAID DEVICE**

(71) Applicant: Zobele Holding SpA, 38123 Trento (IT)
(72) Inventor: DEFLORIAN, Stefano, 38123 TRENTO (IT); SORDO, Walter, 38123 Trento (IT); MORHAIN, Cedric, 08019 BARCELONA (ES)
(74) Representative: Herrero & Asociados, S.L.

(57) **Abstract**

The device comprises a container (1) defining a cavity for housing a liquid with volatile substances, and a cover (2) fixed to the container (1), said cover (2) comprising: a membrane (21), a barrier layer (22), that is removed before a first use of the device; an intermediate layer (24) that is placed between the membrane (21) and the barrier layer (22); and a peelable tab (25) defining a fixing area (3), which is an area where the cover (2) is fixed to the container (1) between the peelable tab (25) and the rest of the cover (2), wherein the membrane (21) comprises a gap (26) or a reduced thickness portion at the fixing area (3) or the membrane (21) ends at the fixing area (3).

It permits to remove a barrier layer before a first use, enhancing the opening and maintaining a membrane welded to the container.

## Description

### Field of the invention

The present invention relates to a device for diffusing volatile substances and to a method for manufacturing said device.

### Background of the invention

Membranes have been used since many years in devices for diffusing volatile substances, such as air fresheners and pest control devices.

In these devices, the membrane closes a container that is filled with a liquid with the volatile substances and let only the vapors to be diffused, but the liquid cannot exit the container.

Since the early polyethylene monolithic membranes, made of a thin wall of plastic without any pores created therein, several new solutions have been proposed to improve performance.

Now, the technology of membrane is not only about the membrane, but about a complete solution that comprises:
- A membrane that allows the vapor to be diffused, but not a liquid that contains the volatile substances;
- A container on which the membrane can be fixed;
- An activation system, consisting in a barrier layer, that closes the system between manufacturing and a first use, but can be removed before a first use.

A basic solution comprises a multilayer film, with the latest layer being a very thin (about 50 microns) layer of polyethylene, and the rest being built around an aluminum barrier layer, with one layer of polyester to reinforce the aluminum, and one layer to achieve the pealing from the polyethylene layer when pulling on it.

This multilayer is welded on a thermoformed cup made with a polyethylene internal layer, with a tab outside this cup.

When user pulls the tab, the very thin polyethylene layer breaks at the level of the sealing, leading to the final results, that on the tab side, the polyethylene is remaining on that multilayer structure and on the container side, the polyethylene detached from the multilayer remains alone on top of the container.

Using this kind of multilayer film provides several advantages in comparison if the different layers were applied sequentially in the container production process. On one side, the container is made with only two reels, one from the bottom side and another one, the multilayer with membrane, for the top side. Another advantage is that the adhesion between the different layers can be easily controlled as the multilayer is done in a continuous and stable process such as lamination or coextrusion.

However, the use of a polyethylene membrane has the problem that it is limited in performance, as it is based on permeability and then it has a quite high selectivity and may separate the components of a fragrance.

One big improvement for olfactive performance is the use of a membrane, i.e., a plastic film in which a certain porosity has been created. Nevertheless, the microporosity of these membranes reduces the amount of plastic and a membrane with a same thickness than the standard thin polyethylene will have a very poor mechanical resistance, maybe poor enough to break when user pulls the barrier tab, and poor enough to be easily perforated by user fingers during product life.

Therefore, membranes applied to devices for diffusing volatile substances have higher thickness (e.g., 80, 100, even in some cases up to 300 microns). With such high thickness, it become quite challenging that the membrane layer breaks when user remove the aluminum.

### Description of the invention

Therefore, an objective of the present invention is to provide a device for diffusing volatile substances and its manufacturing method that permits to remove a barrier layer of the device before a first use, enhancing this opening and maintaining a membrane welded to the container.

With the device and method of the invention, the aforementioned drawbacks are resolved, presenting other advantages that will be described below.

The device for diffusing volatile substances and the manufacturing method according to the present invention are defined in the independent claims. Additional optional features are included in the dependent claims.

In particular, the device for diffusing volatile substances comprises a container defining a cavity for housing a liquid with volatile substances, and a cover fixed to the container, said cover comprising:
- a membrane,
- a barrier layer, that is removed before a first use of the device;
- an intermediate layer that is placed between the membrane and the barrier layer; and
- a peelable tab defining a fixing area, which is an area where the cover is fixed to the container between the peelable tab and the rest of the cover,
wherein the membrane comprises a gap or a reduced thickness portion at the fixing area, or the membrane ends at the fixing area.

As the function of this gap in the membrane is to avoid to have a thick layer that will prevent the separation of the barrier layer from the membrane, this gap could also be partial, i.e., a reduced thickness portion, that in the area of the gap, the membrane has not been completely removed but only in a fraction of its thickness. The reduction in the thickness will be depending on the mechanical resistance of the material per se.

Thanks to this feature, the barrier layer can be removed by the peelable tab not damaging the membrane.

Furthermore, the container comprises a flange, the cover being fixed to the flange of the container, and the container is preferably made from polyethylene terephthalate and polypropylene.

According to a preferred embodiment, the intermediate layer is made from polyethylene, so that it is fixed to the container, e.g., by welding, to the polypropylene of the container.

For reinforcing the barrier layer, the cover can also comprise a reinforcing layer in contact with said barrier layer.

According to two alternatives embodiments, the peelable tab is an extension of the barrier layer and the intermediate layer, or the peelable tab is an extension of the barrier layer, the intermediate layer, and the membrane, the membrane comprising the gap or reduced thickness portion for the direct fixation of the intermediate layer to the container.

Preferably, the peeling tab devoid of membrane or the gap or the reduced thickness portion has a width that is the same as the width of the cavity of the container, and a length between 1 mm up to the same length of the peeling tab.

The method for manufacturing a device for diffusing volatile substances as defined previously comprises the following steps:
- thermoforming a container;
- filling the container with volatile substances;
- sealing the container by applying a cover, said cover comprising a membrane and a barrier layer; and
- removing a least partially the membrane in a fixing area, which is an area where the cover is fixed to the container between a peelable tab and the rest of the cover.

Preferably, the membrane is removed by a laser.

Furthermore, the sealing is preferably made by a sealing tool, and during sealing pressure is applied only on the fixing area.

Still preferably, the sealing tool is applying pressure and heat in an area that, on one side, goes beyond the area where membrane has been cut or removed and, on the other side, does not apply pressure in the area corresponding to the thermoformed cavity.

According to a preferred embodiment the sealing tool has a radius of at least 0.5 mm on its side close to the cavity of the container.

### Brief description of the drawings

For a better understanding of what has been stated, some drawings are attached in which, schematically and only as a non-limiting example, a practical case of embodiment is represented.
Figure 1 is a diagrammatical section of the device for diffusing volatile substances according to the present invention; and
Figures 2 and 3 are two alternative embodiments of the device for diffusing volatile substances according to the present invention during its manufacturing, showing also a welding tool.

### Description of a preferred embodiment

The device for diffusing volatile substances according to the present invention comprises a container 1 with a cavity containing a liquid with volatile substances, said container 1 comprising a flange 11 on which a cover 2 is applied.

The container 1 can be thermoformed cup, e.g., from polyethylene terephthalate (PET) and polypropylene (PP), where PET is giving main consistency of the part and PP is for welding.

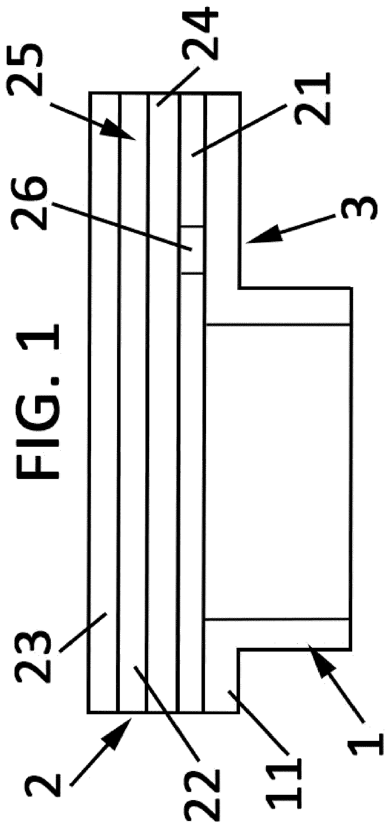

The cover 2 comprises the following layers, as shown in Fig. 1:
- a membrane 21, e.g., of polypropylene, of a thickness of, e.g., 100 microns;
- a barrier layer 22, e.g., made from aluminum, that provides barrier properties; and
- a reinforcing layer 23, e.g., made from polyethylene terephthalate (PET) that mechanically reinforces the barrier layer 22 to withstand a pealing action before a first use;
- an intermediate layer 24, placed between the membrane 21 and the barrier layer 22, preferably made from polyethylene, because polyethylene is weldable to polypropylene, but it does not show a very high adhesion on it, i.e., it is peelable from polypropylene.

Other layers, not shown in Fig. 1, can be added between the intermediate layer 24 and the barrier layer 22. For example, a printed layer to increase aesthetics or to give a message to customer, or also a preliminary barrier layer, e.g., made from Etilen-Vinil-Alcohol (EVOH) to improve chemical resistance of the barrier layer 22 adhesion.

Alternatively, the peelable adhesion between intermediate layer 24 and the rest of the layers of the cover 2 can be achieved by adhesive.

The container 1 is made on a form fill seal machine, where the container 1 is formed, filled with the liquid containing the volatile substances, and the cover 2 is welded on the flange 11 of the container 1, and then container 1 is cut from reels.

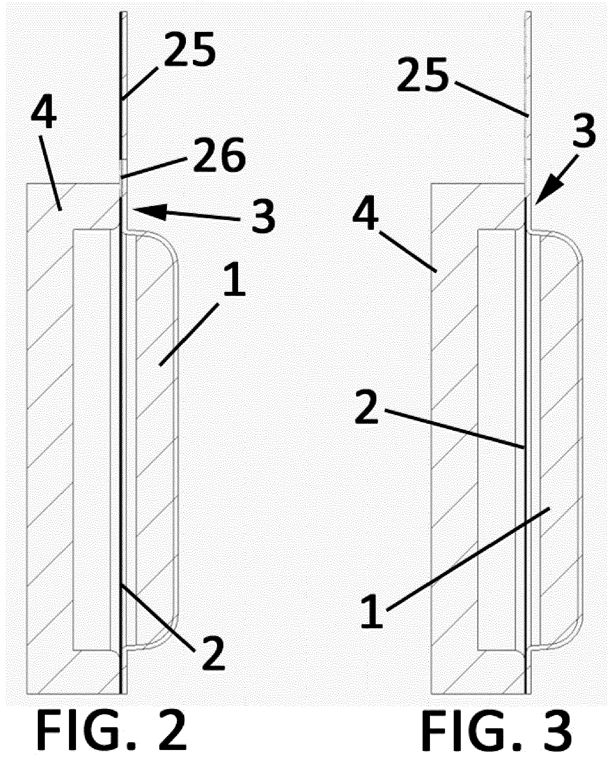

Once cut, the container 1 defines a cavity filled with liquid and the cover 2 comprises peeling tab 25, shown in Fig. 2. This peeling tab 25 can be an extension of the cover 2 or an additional piece that is welded to the cover 2.

Furthermore, the membrane 21 does not extend at least partially on the peeling tab 25. As shown in Fig. 2, the membrane 21 does not extend on the peeling tab 25, or as shown in Fig. 3, the membrane 21 comprises a gap 26 between the peeling tab 25 and the rest of the cover 2. It must pointed out that the gap 26 can also be a reduced thickness portion because it provides the same technical effect.

During the manufacturing of the device according to the present invention, the membrane 21 is removed, for example, using a laser, at least in a fixing area 3 that corresponds to the area between the peeling tab 25 and the rest of the cover 2.

The part in which the membrane 21 has been removed, corresponding to the peeling tab 25 or the gap 26, preferably has the following dimensions:
- a length of minimum 1 mm; and
- a width at least equivalent to the width of the cavity of the container 1.

Regarding its length, its value can be between 1 mm up to the same length of the peeling tab 25.

Regarding its width, its value can be between the width of the cavity of the container 1 and the width of the peeling tab 25.

Considering that this modification (removing a part of the membrane 21) of the cover 2 must be done before welding it to the flange 11 of the container 1, it is important that this modification is done in the right place on the reel to coincide with the right place of welding.

To show that the gap 26 or the end of the membrane 21 coincides with the welding zone 3 between the cover 2 and the container 1, in Figs. 2 and 3 a welding tool 4 is shown.

It must be pointed out that the dimensions cited previously are only non-limitative example, considering that the dimensions could be slightly higher that what defined above, to compensate the lack of precision of positioning between the layers.

This way, when the cover 2 is welded on the container 1, the membrane 21 is welded on polypropylene part of the container 1 in a non-peelable way, but in the peeling tab 25 area, it is the intermediate layer 24 of cover 2 that is welded on the container 1, and this is peelable.

Despite the fact that reference has been made to a specific embodiment of the invention, it is clear to a person skilled in the art that the described device is susceptible to numerous variations and modifications, and that all the mentioned details can be substituted by others technically equivalent ones, without departing from the scope of protection defined by the appended claims.

## Claims

1. Device for diffusing volatile substances, comprising a container (1) defining a cavity for housing a liquid with volatile substances, and a cover (2) fixed to the container (1), said cover (2) comprising:
- a membrane (21),
- a barrier layer (22), that is removed before a first use of the device;
- an intermediate layer (24) that is placed between the membrane (21) and the barrier layer (22); and
- a peelable tab (25) defining a fixing area (3), which is an area where the cover (2) is fixed to the container (1) between the peelable tab (25) and the rest of the cover (2), **characterized in that** the membrane (21) comprises a gap (26) or a reduced thickness portion at the fixing area (3) or the membrane (21) ends at the fixing area (3).

2. Device for diffusing volatile substances according to claim 1, wherein the container (1) comprises a flange (11), the cover (2) being fixed to the flange (11) of the container (1).

3. Device for diffusing volatile substances according to claim 1 or 2, wherein the container (1) is made from polyethylene terephthalate and polypropylene.

4. Device for diffusing volatile substances according to any one of the previous claims, wherein the intermediate layer (24) is made from polyethylene.

5. Device for diffusing volatile substances according to any one of the previous claims, wherein the cover (2) also comprises a reinforcing layer (23) in contact with the barrier layer (22).

6. Device for diffusing volatile substances according to any one of the previous claims, wherein the peelable tab (25) is an extension of the barrier layer (22) and the intermediate layer (24).

7. Device for diffusing volatile substances according to any one of claims 1-5, wherein the peelable tab (25) is an extension of the barrier layer (22), the intermediate layer (24), and the membrane (24), the membrane (24) comprising the gap (26) or the reduced thickness portion for the direct fixation of the intermediate layer (24) to the container (1).

8. Device for diffusing volatile substances according to any one of the previous claims, wherein the peeling tab (25) devoid of membrane (24) or the gap (26) or reduced thickness portion has a width that is the same as the width of the cavity of the container (1).

9. Device for diffusing volatile substances according to any one of the previous claims, wherein the portion of the peeling tab (25) devoid of membrane (24) or the gap (26) or reduced thickness portion has a length between 1 mm up to the same length of the peeling tab (25).

10. Method for manufacturing a device for diffusing volatile substances according to any one of the previous claims, **characterized in that** it comprises the following steps:
- thermoforming a container (1);
- filling the container (1) with volatile substances;
- sealing the container (1) by applying a cover (2), said cover (2) comprising a membrane (21) and a barrier layer (22); and
- removing a least partially the membrane (21) in a fixing area (3), which is an area where the cover (2) is fixed to the container (1) between a peelable tab (25) and the rest of the cover (2).

11. Method according to claim 10, wherein the membrane (2) is removed by a laser.

12. Method according to claims 10 or 11, wherein the sealing is made by a sealing tool, and during sealing pressure is applied only on the fixing area (3).

13. Method according to claim 12, wherein the sealing tool has a width of at least 0.5 mm on its side close to the container (2).
